(19) Europäisches Patentamt
European Patent Office
Office européen des brevets

(11) Numéro de publication : **0 314 593 B1**

(12) **FASCICULE DE BREVET EUROPEEN**

(45) Date de publication du fascicule du brevet :
14.08.91 Bulletin 91/33

(51) Int. Cl.⁵ : **A61F 2/42, A61B 17/56**

(21) Numéro de dépôt : **88450037.2**

(22) Date de dépôt : **26.10.88**

(54) **Implant prothétique articulaire à fixation temporaire.**

(30) Priorité : 27.10.87 FR 8715024

(43) Date de publication de la demande :
03.05.89 Bulletin 89/18

(45) Mention de la délivrance du brevet :
14.08.91 Bulletin 91/33

(84) Etats contractants désignés :
AT BE CH DE ES GB GR IT LI LU NL SE

(56) Documents cités :
EP-A- 0 034 912
DE-U- 8 533 557
US-A- 3 809 075

(73) Titulaire : **Barouk, Louis Samuel**
**La Manchotte - Yvrac**
**F-33370 Tresses (FR)**

(72) Inventeur : **Barouk, Louis Samuel**
**La Manchotte - Yvrac**
**F-33370 Tresses (FR)**

(74) Mandataire : **Thébault, Jean-Louis**
**Cabinet Thébault S.A. 1 Allées de Chartres**
**F-33000 Bordeaux (FR)**

## Description

La présente invention a trait à un implant prothétique articulaire destiné à la chirurgie orthopédique.

De nombreux implants prothétiques articulaires sont déjà utilisés en chirurgie orthopédique et sont destinés à remplacer les surfaces articulaires altérées. Ils sont constitués en général d'une ou deux parties prothétiques mises en place entre les deux pièces devant rester mobiles l'une par rapport à l'autre (voir, par exemple, DE-U-8533557).

La fixation aux pièces osseuses de ces deux parties prothétiques se fait à titre définitif par l'intermédiaire de tiges ou d'aspérités solidaires de chacune des deux parties prothétiques et pénétrant l'une des deux pièces osseuses, ou les deux, sans ou avec l'intermédiaire de ciment chirurgical, par exemple du méthylacrylate de méthyle, ou par tout autre mode de fixation.

Le but de l'invention est de proposer dans le cas de prothèses articulaires métatarso ou métacarpo phalangiennes, une nouvelle technique de fixation de tels implants prothétiques assurant, d'une part, un positionnement rigoureux de l'implant, et, d'autre part, l'absence d'ancrage prothétique définitif dans les pièces osseuses.

A cet effet, l'invention a pour objet un implant prothétique articulaire à fixation temporaire, plus particulièrement destiné aux pieds et aux mains, constitué, d'une part, d'une cupule ou analogue, percée en son centre d'un trou et destinée à être mise en place entre deux pièces osseuses au droit de leur articulation et, d'autre part, d'une broche de fixation, amovible, susceptible de coulisser librement dans le trou de ladite cupule, destinée à immobiliser provisoirement la cupule et les pièces osseuses adjacentes en étant enfilée au travers des canaux médullaires des pièces osseuses intéressées et du trou de la cupule préalablement mise en place.

Un tel dispositif assure un centrage parfait de la cupule vis à vis des pièces osseuses adjacentes le temps de la reconstitution des tissus fibreux périprothétiques (en général 4 semaines) et permet, à l'issue de ce délai, de retirer aisément et sans intervention chirurgicale la broche d'immobilisation :

La cupule retrouve ainsi sa liberté de mouvement vis à vis des deux pièces osseuses en regard tout en étant parfaitement maintenue en place.

D'autres caractéristiques et avantages ressortiront de la description qui va suivre d'un mode de réalisation de l'objet de l'invention, description donnée à titre d'exemple uniquement et en regard du dessin annexé sur lequel :

— Figure 1 représente une vue en élévation frontale d'une prothèse cupule conforme à l'invention ;

— Figure 2 est une vue en coupe transversale axiale suivant la ligne II-II de la cupule de la figure 1 ;

— Figure 3 est une vue en élévation d'une broche selon l'invention, et

— Figure 4 illustre la cupule et sa broche d'immobilisation en place dans un orteil.

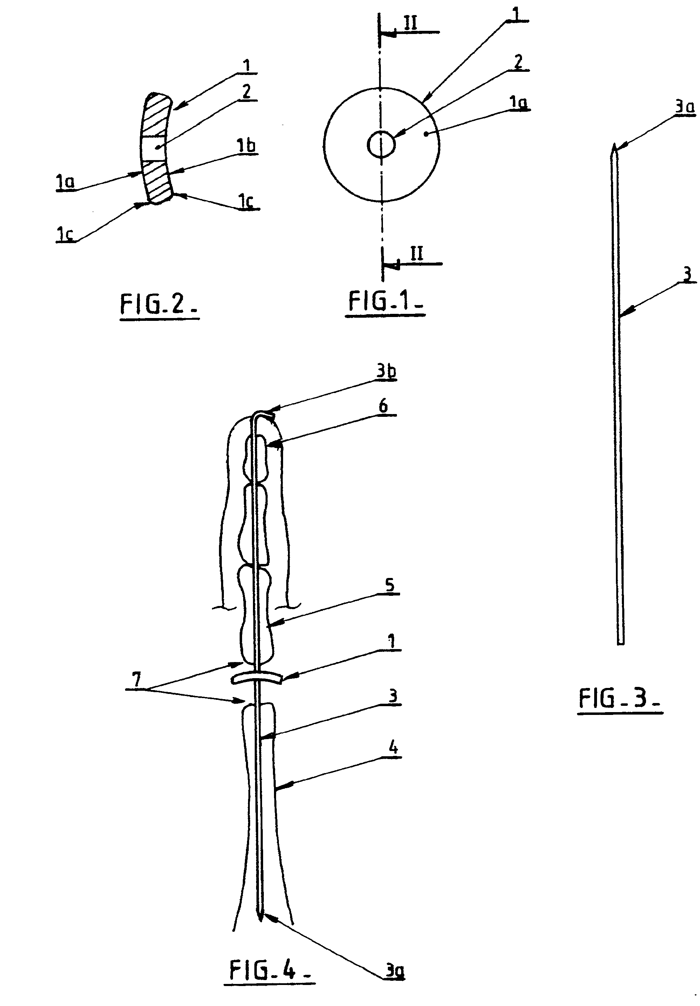

Sur les figures 1 et 2 on a représenté un implant selon l'invention constitué d'une cupule circulaire 1 de préférence à faces parallèles respectivement convexe 1a et concave 1b, réalisée en un matériau susceptible d'être bien toléré par les tissus vivants, par exemple l'acier inoxydable, le titane, la céramique, etc... Le diamètre de la cupule 1 peut bien entendu varier en fonction des articulations métatarso ou métacarpo phalangiennes à traiter, par exemple entre 8 et 18 mm dans la plupart des cas.

L'épaisseur de la cupule 1 est relativement importante et de l'ordre de 2 à 5 mm environ. La courbure des faces 1a et 1b est légère et correspond sensiblement à celle des surfaces articulaires en contact.

Les arêtes circulaires périphériques 1c sont légèrement émoussées.

La cupule est percée axialement en son centre d'un trou 2 d'un diamètre de l'ordre du millimètre ou légèrement plus, afin de permettre le libre passage d'une broche de fixation temporaire illustrée en 3 sur la figure 3.

La broche 3 est cylindrique, de diamètre légèrement inférieur à celui du trou 2 de la cupule 1 et de préférence en un matériau tel que l'acier inoxydable permettant son coudage et son sectionnement à la longueur désirée.

Sur la figure 4, on a représenté un orteil muni d'un implant selon l'invention. La cupule 1 est interposée entre le métatarsien 4 et la première phalange 5 et est maintenue en place par la broche 3 engagée dans le canal médullaire des trois phalanges et du métatarsien, l'extrémité 3a étant fichée dans la partie osseuse distale du métatarsien 4.

L'autre extrémité de la broche 3 dépasse au bout de la dernière phalange 6 et est recourbée en 3b contre l'extrémité de ladite phalange.

La mise en place de l'ensemble s'opère de la manière suivante.

L'articulation à traiter est incisée et nettoyée. A ce propos, on peut enlever, si nécessaire, comme représenté sur la figure 4 en 7, les parties osseuses en regard altérées. La broche 3 est alors introduite dans le canal médullaire des phalanges successives, se dirigeant vers la partie distale de l'orteil : la broche fait alors issue à l'extrémité de l'orteil ; puis, la broche est dirigée vers la partie proximale, successivement introduite dans le trou central 2 de la cupule 1 et, enfin, dans le canal médullaire du métatarsien 4.

La broche est alors sectionnée à son extrémité distale de façon à laisser dépasser hors de l'orteil une petite longueur de broche qui sera recourbée pour former l'extrémité 3b.

Une telle immobilisation par transfixion de la cupule 1 permet, comme on peut l'observer sur la figure 4, de positionner parfaitement la cupule à la fois dans l'axe des pièces osseuses intéressées 4 et 5 et à la distance désirée vis à vis des extrémités en regard desdites pièces osseuses, afin de laisser la place à la régénération à la fois des tissus osseux et de la fibrose assurant le maintien en place de la cupule 1 et une bonne surface de friction vis à vis de celle-ci.

La broche 3 est laissée en place le temps nécessaire à la régénération des tissus environnants la cupule, par exemple un mois, puis enlevée très facilement par traction sur l'extrémité recourbée 3b sans avoir à intervenir chirurgicalement. La broche retirée laisse la cupule 1 interposée en bonne place entre les deux pièces osseuses adjacentes sans risques de descellement ou d'érosion osseuse observés dans les prothèses connues.

L'articulation ainsi appareillée récupère une complète mobilité puisqu'il ne subsiste aucune fixation ou entrave entre la cupule 1 et les deux pièces osseuses adjacentes.

Enfin, l'invention n'est évidemment pas limitée au mode de réalisation représenté et décrit ci-dessus mais en couvre au contraire toutes les variantes notamment en ce qui concerne les formes et dimensions de la cupule 1 et de la broche 3, ainsi que la nature du matériau constitutif de celles-ci. C'est ainsi que les faces 1a et 1b de la cupule 1 sont de préférence parallèles mais elles pourraient avoir des courbures légèrement différentes.

De même, l'implant selon l'invention peut être mis en place au droit de l'une quelconque des articulations d'un doigt ou d'un orteil, voire plusieurs articulations d'un même doigt ou orteil.

## Revendications

1. Implant prothétique articulaire à fixation temporaire, plus particulièrement destiné aux pieds et aux mains, constitué, d'une part, d'une cupule ou analogue percée en son centre d'un trou (2) et destinée à être mise en place entre deux pièces osseuses (4, 5) au droit de leur articulation et, d'autre part, d'une broche (3) de fixation, amovible, susceptible de coulisser librement dans le trou (2) de ladite cupule (1), et destinée à immobiliser provisoirement la cupule (1) et les pièces osseuses adjacentes (4, 5) en étant enfilée au travers des canaux médullaires des pièces osseuses intéressées (4, 5, 6) et du trou (2) de la cupule (1) préalablement mise en place.

2. Implant suivant la revendication 1, caractérisé en ce que les faces de ladite cupule (1) sont parallèles, l'une (1a) étant convexe, l'autre (1b) concave.

## Claims

1. Prosthetic joint implant with temporary fastening, more particularly adapted for the feet and hands, formed, on the one hand, by a cupula or the like whose centre is drilled with a hole (2) and which is adapted to be positioned between two bone sections (4, 5) at the level of their articulation and, on the other hand, by a detachable securing pin (3) which may freely slide in the hole (2) of the cupula (1) and is adapted provisionally to immobilize the cupula (1) and the adjacent bone sections (4, 5) while being threaded through the medullary canals of the bone sections (4, 5, 6) involved and the hole (2) of the previously positioned cupula (1).

2. Implant as claimed in claim 1, characterized in that the surfaces of the cupula (1) are parallel, one (1a) being convex and the other (1b) concave.

## Patentansprüche

1. Prothetisches Gelenkimplantat für zeitweilige Fixierung, insbesondere für Füße und Hände bestimmt, bestehend aus einerseits einem Napf oder dergleichen, der in seinem Zentrum von einem Loch (2) durchsetzt ist und zwischen zwei Knochenteilen (4, 5) am Ort ihres Gelenks plazierbar ist, und andererseits einer entfernbaren Fixierungsnadel (3), die frei gleitbeweglich in dem Loch (2) des Napfes (1) ist und zum provisorischen Festlegen des Napfes (1) und der anschließenden Knochenteile (4, 5) bestimmt ist, indem sie durch die Markkanäle der betreffenden Knochenteile (4, 5, 6) und das Loch (2) des vorher plazierten Napfes (1) gefädelt wird.

2. Implantat nach Anspruch 1, dadurch gekennzeichnet, daß die Flächen des Napfes (1) parallel sind, wobei eine (1a) konvex ist und die andere (1b) konkav ist.

FIG.2.

FIG.1.

FIG.3.

FIG.4.